# DEMANDE DE BREVET EUROPEEN

(11) **EP 0 708 071 A1**
(43) Date de publication de la demande: **24.04.1996**
(21) Numéro de dépôt: 95402175.4
(22) Date de dépôt: 28.09.1995
(51) Int. Cl.: C07C 13/15, C07C 2/86

(54) **Procédé et dispositif de préparation des 5-(alkylalkylidène)-1,3-cyclopen-tadiènes**

(30) Priorité: 03.10.1994 FR 9411799
(71) Demandeur: ELF ANTAR FRANCE, F-92400 Courbevoie (FR)
(72) Inventeur: Chaffardon, Alain, F-69001 Lyon (FR); Roman, Jean-Philippe, F-69440 Mornant (FR)
(74) Mandataire: Boillot, Marc

(57) **Abrégé**

Le procédé de préparation des 5-(alkylalkylidène)-1,3cyclopentadiènes consiste à mélanger du cyclopentadiène avec une alkylcétone et à faire réagir le mélange sur une résine anionique fortement basique contenant de 25 à 55 % d'eau.

## Description

La présente invention vise un procédé de préparation des 5-(alkylalkylidène)-1,3-cyclopentadiènes et le dispositif de mise en oeuvre.

Ces composés sont particulièrement recherchés comme additif ou composant dans les carburants, notamment pour leur effet anticliquetis. Cependant, il existe bon nombre de modes de préparation de ces composés, appelés encore dialkylfulvènes, en particulier pour le 6,6-diméthylfulvène.

Le brevet US 3.192.275 décrit un mode de préparation des dialkylfulvènes qui consiste à faire réagir le cyclopentadiène avec un composé alkylacétonique en présence d'une alkylamine en milieu liquide. Le procédé est discontinu et comprend plusieurs étapes dont une étape de réaction, plusieurs étapes de séparation des phases aqueuses et organiques comme la décantation et la rectification. Bien que les rendements soient très bons, ce procédé présente de nombreux inconvénients liés à l'utilisation de volumes importants d'alkylamines comme agent condensant non récupérable, et au nombre élevé d'opérations unitaires. Il est d'ailleurs connu qu'il est préférable d'utiliser des catalyseurs solides facilement isolables et récupérables, pour les fabrications de produits à l'échelle industrielle.

Parmi les procédés de fabrication des alkylfulvènes correspondant à un procédé de catalyse hétérogène c'est-à-dire utilisant un catalyseur solide, le brevet US 2.589.969 décrit la fabrication du diméthylfulvène par réaction du dicyclopentadiène avec de l'acétone en présence de bauxite activée à une température comprise entre 200 et 480°C. Le brevet Fr 1.134.170 et G.H. Mc Cain (Organic Chem., 1957, vol.23,632-633) décrivent la préparation du diméthylfulvène à partir de dérivés du cyclopentadiène et d'acétone en présence d'une résine échangeuse d'ions anioniques déshydratée. Dans ces procédés, de grandes quantités de résidus résineux inutiles sont obtenus comme sous-produits et le rendement en dialkylfulvène est en moyenne compris entre 50 et 75 % poids.

Le but de la présente invention est de disposer d'un procédé susceptible de préparer en grande quantité à l'échelle industrielle des dialkylfulvènes par catalyse hétérogène avec un rendement supérieur à 90 %, afin d'éviter de multiplier le nombre d'opérations unitaires indépendantes.

La présente invention a donc pour objet un procédé par catalyse hétérogène de préparation des 5-(alkylalkylidène)-1,3-cyclopentadiènes consistant à faire réagir du cyclopentadiène avec une alkylcétone en présence d'une résine échangeuse d'ions fortement basique caractérisé en ce que la dite résine contient entre 25 et 55 % en poids d'eau.

On a en effet constaté que de façon inattendue, la réaction du cyclopentadiène sur l'acétone était grandement améliorée par la présence d'une quantité déterminée d'eau dans la résine, puisqu'à moins de 25 % et à plus de 55 % d'eau, les rendements chutaient à moins de 80 %.

Ces résines échangeuses d'ions sont de préférence des résines anioniques basiques de structure type gel ou macroréticulé, constituées par un polymère styrène/divinylbenzène. L'échange d'ions est assuré par des fonctions hydroxydes d'ammonium procurant à la résine une basicité comprise entre 0.05 et 1.2 équivalents molaires d'ions hydroxydes par mole de cyclopentadiène. Les résines préférées de l'invention sont les résines ayant une basicité comprise entre 0.06 et 1. équivalent molaire d'ions hydroxydes par mole de cyclopentadiène.

Cependant, les résines de ce type usuellement disponibles dans le commerce ont des teneurs en eau comprises entre 60 et 75 % poids de la résine. Aussi dans le cadre la présente invention, ces résines du commerce sont soit séchées partiellement, par exemple dans une enceinte chauffée entre 25 et 60°C pendant un temps suffisant, dépendant de la teneur initiale en eau de ladite résine, soit hydratées de façon immédiate par trempage dans une quantité suffisante d'eau, ces deux modes permettant d'ajuster la teneur en eau de la résine à l'intervalle revendiqué.

Pour le procédé selon l'invention, il est impératif d'ajuster cette teneur en eau de la résine pour à la fois assurer la mobilité des ions et ne pas perturber la cinétique de réaction de condensation du cyclopentadiène sur l'alkylcétone.

Selon l'invention, le procédé comprend au moins une étape de réaction, au moins une étape de séparation des produits de la réaction et éventuellement au moins une étape de régénération de ladite résine échangeuse d'ions. Ladite étape de réaction consiste à mettre en contact progressivement la résine à teneur en eau ajustée selon l'invention, baignée dans un solvant organique, l'ensemble étant maintenu à une température comprise entre -20°C et 60°C, avec un mélange homogène de cyclopentadiène et d'au moins un alkylcétone obtenu dilué ou non dans ledit solvant organique, le rapport molaire alkylcétone/cyclopentadiène étant compris entre 0.6 et 1.76.

Dans un mode préféré du procédé, le solvant organique est choisi dans le groupe constitué par les alcanes, les cycloalcanes et les isoalcanes comprenant de 5 à 8 atomes de carbone, et les dérivés benzéniques tels que le toluène, le xylène ou leurs dérivés pris seuls ou en mélange.

Le rapport molaire alkylcétone/cyclopentadiène est de préférence compris entre 0.82 et 1.26 pour un rendement en dialkylfulvène supérieur à 90 %.

L'étape de séparation des produits de réaction consiste à extraire les dialkylfulvènes du solvant organique par une méthode simple connue en soit telle que la distillation ou la seule évaporation dudit solvant organique utilisé.

L'étape de régénération de ladite résine échangeuse d'ions consiste à laver celle-ci abondamment avec le dit solvant organique de façon à éliminer toutes traces de réactif, puis à la rincer avec de l'acétone, et enfin à l'hydrater à la teneur revendiquée par lavage à l'eau ces opérations étant conduites à une température voisine de la température ambiante.

Selon un premier mode de réalisation de l'invention, ces étapes sont réalisées de façon discontinue. Dans ce mode de réalisation, l'étape de réaction consiste à introduire ledit mélange homogène de réactifs dans un bain de résine, dans lequel le volume de solvant organique correspond de 0 à 10 fois le volume de la résine échangeuse d'ions, puis à maintenir ce bain sous agitation pendant au moins 30 minutes et au plus 3 heures entre -5°C et 40°C, enfin à évacuer le solvant de réaction contenant les dialkylfulvènes et à rincer ladite résine 3 à 5 fois par un volume de solvant organique correspondant entre 0.5 et 1.5 fois le volume de résine.

Les dialkylfulvènes sont récupérés en solution dans le mélange constitué par le solvant de réaction et des solvants de rinçage de la résine, puis ils sont séparés par tout procédé connu de l'homme du métier comme l'évaporation, la rectification et la distillation pendant l'étape de séparation.

Selon un deuxième mode de réalisation de l'invention, ces étapes s'enchaînent de façon continue. Au cours de l'étape de séparation, le mélange homogène de cyclopentadiène et d'alkylcétone dilué dans le solvant organique selon l'invention, dont le volume est au plus cinq fois le volume cumulé des réactifs, circule sur la résine échangeuse d'ions à une température comprise entre -5 et 40°C avec une vvh (volume de charge sur volume de résine par heure) comprise entre 1,05 et 6,75 et à une pression comprise entre 0,015 et 8 bars.

On ne sortirait pas du cadre de la présente invention en renouvelant les passages du fluide résultant de la première étape de réaction et de le faire recirculer sur la résine de cette première étape de séparation ou sur une résine identique au cours d'une seconde étape de séparation dans un deuxième réacteur placé à la sortie du premier.

Après les étapes de réaction successives, les dialkylfulvènes sont séparés du solvant organique et des traces d'alkylcétone et de cyclopentadiène par passage dans un réacteur de séparation mettant en oeuvre un procédé connu de l'homme du métier comme par exemple l'évaporation ou la distillation.

Pour le deuxième procédé selon l'invention on peut avantageusement remplacer le cyclopentadiène pur par une coupe dite C5 d'essence dite de vapocraquage récupérée en sortie d'une unité de vapocraquage, l'additivation se faisant in situ dans la coupe récupérée.

Un autre objet de l'invention est le dispositif de mise en oeuvre du procédé selon l'invention qui, quel que soit la continuité du procédé comprend au moins un réacteur pour l'étape de réaction comprenant des moyens de chauffage et de régulation des flux entrant ou sortant, au moins un dispositif de séparation du type évaporateur ou distillation et éventuellement un réacteur séparé pour la régénération de la résine usée.

Pour le dispositif de mise en oeuvre du procédé continu, il peut être avantageux de disposer de deux circuits de réaction avec des réacteurs fonctionnant en parallèle et alternativement soit en réaction pour fabriquer les dialkylfulvènes, soit en régénération pour régénérer la résine usée.

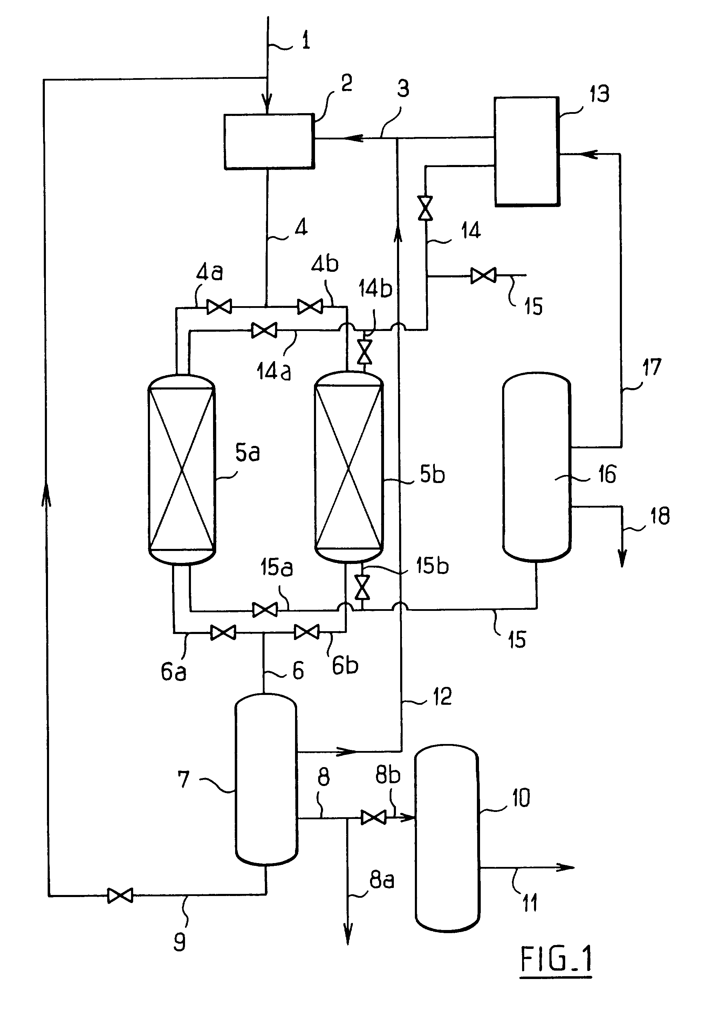

Afin d'expliciter le procédé selon l'invention, la figure 1 présente une illustration du procédé et du dispositif continu de préparation des dialkylfulvènes.

La charge de cyclopentadiène arrive via la conduite 1 dans un mélangeur 2 dans lequel est également envoyé via la conduite 3 l'alkylcétone stockée dans le réservoir 13. Après mélange, les réactifs sont dirigés via la conduite 4 vers un réacteur contenant la résine échangeuse (5a ou 5b). Le mélange après réaction est envoyé via les conduites 6a ou 6b et 6 vers un évaporateur 7, le cyclopentadiène résiduel pouvant être recyclé via la conduite 9 vers la conduite 1 et l'acétone via la conduite 12 vers la conduite 3. Le dialkylfulvène est récupéré via la conduite 8 afin d'être stocké ou utilisé directement (via la conduite 8a) ou envoyé dans une colonne de distillation 10 pour y être purifié et recueilli via la conduite 11.

Les deux réacteurs 5a et 5b fonctionnent en parallèle mais l'un en réaction, l'autre en régénération. Si le réacteur 5a est utilisé pour la fabrication de dialkylfulvène, les conduites 4b et 6b sont fermées au moyen des deux vannes représentées. Par contre, les vannes du circuit de régénération des conduites 14b et 15b de part et d'autre du réacteur 5b sont ouvertes tandis que celles des conduites 14a et 15a menant au réacteur 5a sont fermées.

La régénération consiste à laver intérieurement le réacteur 5b par de l'acétone venant du réservoir 13 via les conduites 14 et 14b, à évacuer cette acétone de lavage par les conduites 15b puis 15, à purifier l'acétone dans le réacteur 16 et à renvoyer l'acétone pure dans le réservoir 13 via la conduite 17 pour la recycler. Lorsqu'on considère la résine suffisamment lavée, on interrompt l'arrivée d'acétone en obturant la conduite 14 en fermant la vanne qui y est placée et en faisant pénétrer une certaine quantité d'eau dans la conduite 14 via la conduite 15 pour hydrater la résine, puis en isolant le réacteur en fermant les vannes 14b et 15b. La conduite 18 de sortie du séparateur 16 permet d'évacuer les composés autres que l'acétone.

En vue de décrire les avantages de la présente invention, les exemples suivants visent à l'illustrer sans toutefois la limiter.

### EXEMPLE I

Le présent exemple vise à démontrer l'influence d'une quantité limitée d'eau présente dans la résine échangeuse d'ions sur le rendement de la réaction de condensation de l'acétone sur le cyclopentadiène pour la fabrication du diméthylfulvène.

A cet effet, on prépare différents échantillons d'une résine échangeuse d'ions possédant des taux d'hydratation de 2 %(R2), 5 %(R5), 15 %(R15), 25 %(R25), 40 %(R40), 50 %(R50), 55 %(R55), 70 %(R70), 80 %(R80).

On prendra comme résines de départ soit une résine de type gel, IRA 420-OH, commercialisée par Rohm & Haas qui contient 64.3 % en poids d'eau pour les échantillons contenant de 2 à 55 % d'eau, soit une résine de type macroréticulée, AMBERLYST A26-OH/E de teneur initiale en eau de 74,3 % en poids.

Pour chaque échantillon de résine nécessitant un séchage partiel, on verse un kilogramme de résine dans un cristallisoir qu'on place dans une étuve à 40°C. On établit un vide correspondant à une pression de 5 mm de mercure, pendant le temps nécessaire à l'obtention de la teneur en eau désirée, c'est-à-dire pendant 1 à 4 heures. La différence de poids avant et après séchage permet de déterminer la teneur en eau exacte de l'échantillon.

Pour chaque échantillon de résine nécessitant une teneur en eau supérieure à la quantité initiale, on ajoute le volume d'eau nécessaire à la résine en vue d'atteindre la teneur en eau visée, cette eau ajoutée étant immédiatement absorbée par les résines selon l'invention.

L'efficacité de ces échantillons est testée selon la procédure suivante.

Dans un réacteur équipé d'une agitation mécanique, d'un système de condensation, d'un système de contrôle de la température, et d'une ampoule de coulée, on introduit 150 g d'un échantillon de résine précédemment préparé et on agite l'ensemble à une vitesse de 200 tours/mn. On ajoute alors en 21 mn, un mélange à 15°C de 250 g de cyclopentadiène fraîchement distillé et de 265 g d'acétone. On contrôle la température du réacteur pendant l'addition de façon à ce que celle-ci n'excède pas 60°C, et on augmente progressivement l'agitation jusqu'à 500 tours/mn. A la fin de l'addition on agite le mélange pendant 1,5 h, puis on le filtre. La résine est rincée 3 fois par 200 ml d'acétone. Le produit de réaction ainsi que les solvants de rinçage de la résine sont regroupés pour être évaporés à l'évaporateur rotatif à la température maximale de 35°C, et sous un vide correspondant à une pression de 18 mm de mercure. On récupère ainsi une certaine quantité de 6,6-diméthylfulvène qui est pesée pour calculer le rendement en 6,6-diméthylfulvène en % poids par rapport à la charge de cyclopentadiène. Les résultats obtenus pour chaque échantillon de résine sont rassemblés dans le tableau I ci-après.

**TABLEAU I**

| | R2 | R15 | R25 | R40 | R50 | R55 | R70 | R80 |
|---|---|---|---|---|---|---|---|---|
| IRA 420-OH | | | | | | | | |
| %H₂O | 2,1 | 15,2 | 25 | 40,1 | 50,1 | 55 | 70,2 | 80,2 |
| Rendement | 42 | 58 | 83 | 85 | 89 | 82 | 54 | 47 |
| AMBERLYST | | | | | | | | |
| %H₂O | 2,0 | 15,1 | 25,2 | 40 | 50,1 | 55,2 | 69,9 | 80,1 |
| Rendement | 45 | 57 | 90 | 92 | 98 | 96 | 63 | 55 |

Le tableau I montre clairement l'effet de la teneur en H₂O de la résine, les rendements les plus élevés étant obtenus lorsque cette dernière contient de 40 à 55 % d'eau.

D'autre part, on peut observer l'effet de la structure de la résine, l'AMBERLYST A 26-OH/E macroréticulée, apparaissant plus efficace que l'IRA 420-OH, de type gel.

### EXEMPLE II

Le présent exemple vise la préparation du 6,6-diméthylfulvène à partir d'une coupe C5 d'essence de vapocraquage.

Dans une colonne de réaction munie d'une double enveloppe, de 1 m de longueur et de 8 cm de diamètre, on introduit 1,710 kg d'une résine AMBERLYST A 26-OH/E dont la teneur en eau est ajustée à 50 % en poids. Un fluide réfrigérant à 10°C est introduit dans la double enveloppe , et la température interne de la colonne est équilibrée. On introduit alors, à l'aide d'une pompe péristaltique, du cyclohexane préalablement refroidi à 10°C, et ceci à la vitesse de 2,5l/h. Lorsque le lit de résine est humidifié et qu'un flux continu et régulier de cyclohexane sort du réacteur, on introduit, à la vitesse de 1,5 l/h (soit un vvh de 1,75), un mélange préalablement refroidi à 10°C, contenant,
- 5 kg d'une fraction C5 d'essence de vapocraqueur (composée en masse de 29 % de paraffines, 12 % d'aromatiques, 42 % d'oléfines et polyoléfines différentes du cyclopentadiène, 17 % de cyclopentadiène)
- et 732 g d'acétone.

Le débit du fluide réfrigérant circulant à co-courant dans la double enveloppe, est ajusté de manière à ce que la température dans le premier tiers du réacteur n'excède pas 32°C. Au cours de la réaction des échantillons sont prélevés à la sortie du réacteur toutes les 30 mn, et sont analysés en chromatographie en phase gazeuse. On constate que le cyclopentadiène est converti en moyenne à 76 % en 6,6-diméthylfulvène, et que le produit de réaction contient moins de 0,8 % en poids d'acétone n'ayant pas réagi.

Pour améliorer le rendement de cette colonne on peut placer avantageusement au moins une deuxième colonne qui est conditionnée et mise en oeuvre à l'identique de la première. Comme après la première colonne on contrôle le rendement à partir d'échantillons prélevés toutes les trente minutes. On atteint ainsi des rendements supérieurs à 95 %. Si les rendements chutent en dessous de 72 %, généralement après 8 recycles, il est nécessaire de régénérer la résine. On injecte du cyclohexane pendant 1 heure au moyen d'une pompe péristaltique réglée pour un débit de 2,5 l/h pour la laver, puis par de l'acétone pendant 3 h, avec le même débit. On vérifie que sa teneur en eau résiduelle n'excède pas 3,2 % de son poids selon la procédure décrite dans l'exemple I puis on ajoute dans la colonne la quantité d'eau distillée nécessaire pour ramener la teneur en eau de la résine à son niveau initial.

La résine étant régénérée in situ, le réacteur peut être remis en fonctionnement.

Pour éviter l'interruption de la fabrication de diméthylfulvène, on travaille avec deux lignes de réacteurs fonctionnant alternativement et en opposition de phase, l'un pour la fabrication de diméthylfulvène l'autre pour la régénération des résines hors d'usage. Un système de vannes permet de modifier les circuits de façon à isoler les réacteurs pour la régénération de la résine selon les techniques connues en soi.

On passe d'un circuit à l'autre lorsque le rendement en 6,6-diméthylfulvène devient inférieur à 72 %.

Dans le cas présent, l'obtention d'un rendement de 72 % est suffisant car le diméthylfulvène est préparé directement dans l'essence.

Au cours des opérations de régénération, on constate que les résines n'ont subit aucune altération de structure, et conservent leurs caractéristiques mécaniques et leur résistance thermique originales.

## Revendications

1. Procédé de préparation des 5-(alkylalkylidène)-1,3cyclopentadiène consistant à mélanger du cyclopentadiène avec une alkylcétone, à faire réagir le mélange sur une résine anionique fortement basique caractérisé en ce que la résine échangeuse d'ions contient de 25 à 55 % d'eau.

2. Procédé selon la revendication 1 caractérisé en ce que la résine déshydratée présente une basicité comprise entre 0.05 et 1.2 équivalent molaire d'ions hydroxydes disponibles par rapport au cyclopentadiène, et de préférence de 0.05 à 1. équivalent molaire d'ions hydroxydes.

3. Procédé selon l'une des revendications 1 ou 2 caractérisé en ce que la résine échangeuse d'ions est préalablement séchée et/ou hydratée à la teneur en eau voulue.

4. Procédé selon l'une des revendications 1 à 3 caractérisé en ce qu'il comprend successivement au moins une étape de réaction, au moins une étape de séparation et éventuellement une étape de régénération de ladite résine échangeuse d'ions.

5. Procédé selon l'une des revendications 1 à 4 caractérisé en ce que l'étape de réaction consiste à faire réagir sur la résine à teneur en eau ajustée, baignant dans un solvant organique, à une température comprise entre -20°C et 60°C, un mélange de cyclopentadiène et d'au moins une alkylcétone, ledit mélange pouvant être dilué dans le même dit solvant organique, le rapport molaire alkylcétone/cyclopentadiène étant compris entre 0,6 et 1,76, et à recueillir le mélange en solution après réaction.

6. Procédé selon l'une des revendications de 1 à 5 caractérisé en ce que ledit solvant organique appartient au groupe constitué par les alcanes, les cycloalcanes et les isoalcanes comprenant de 5 à 8 atomes de carbone, les dérivés benzéniques tels que le toluène , le xylène ou leurs dérivés pris seuls ou en mélange.

7. Procédé selon l'une des revendications de 1 à 5 caractérisé en ce que le rapport molaire alkylcétone/cyclopentadiène dans le dit mélange est compris entre 0,82 et 1,26.

8. Procédé selon l'une des revendications 1 à 7 caractérisé en ce que l'étape de séparation consiste à séparer les 5-(alkylalkylidène) -1,3 cyclopentadiènes formés des solvants de la réaction et des produits de réaction par évaporation et/ou distillation dudit mélange après réaction.

9. Procédé selon l'une des revendications 1 à 8 caractérisé en ce que pendant l'étape de régénération, ladite résine est lavée par ledit solvant organique, puis rincée avec de l'acétone et enfin hydratée par lavage à l'eau pour obtenir la teneur en eau désirée.

10. Procédé en discontinu selon l'une des revendications de 1 à 9 caractérisé en ce que l'étape de réaction consiste à introduire ledit mélange de réactifs dans un bain de résine, tel que le volume de solvant organique correspond de 0 à 10 fois le volume de la résine, puis à maintenir le bain sous agitation pendant de 30 minutes à 3 heures entre -5°C et 40°C, ensuite, à recueillir le mélange après réaction dilué dans ledit solvant organique et à rincer la résine 3 à 5 fois par un volume de solvant organique correspondant à entre 0,5 et 1,5 fois le volume de la résine et, enfin, à mélanger le solvant de rinçage et le mélange après réaction.

11. Procédé en continu selon l'une des revendications de 1 à 9 caractérisé en ce que l'étape de séparation consiste à faire circuler en continu le mélange de cyclopentadiène et d'alkylacétone, dilués dans le solvant organique dont le volume est au plus cinq fois celui le volume cumulé des réactifs, sur une résine échangeuse d'ions à une température comprise entre -5°C et 40°C à une vvh comprise entre 1,05 et 6,75 et une pression comprise entre 0,015 et 8 bars et en ce que la séparation des 5-(alkylalkylidène)-1,3-cyclopentadiènes du milieu réactionnel est réalisée en continu.

12. Procédé selon la revendication 11 caractérisé en ce que le cyclopentadiène est utilisé pur ou dilué dans une coupe C5 d'essence de vapocraquage.

13. Dispositif de mise en oeuvre du procédé selon l'une des revendications de 1 à 12 caractérisé en ce qu'il comprend au moins un réacteur (5a, 5b) de volume de 1,2 à 1,5 fois supérieur au volume nécessaire de résine pour l'étape de réaction, et au moins un dispositif de séparation pour l'étape de séparation (7), du type évaporateur ou distillation, la régénération de la résine pouvant être faite dans le même réacteur que la réaction ou dans un réacteur parallèle (5a ou 5b), les deux réacteurs pouvant fonctionner alternativement soit en réaction soit en régénération.
